(19) Europäisches Patentamt
European Patent Office
Office européen des brevets

(11) **EP 3 307 366 B1**

(12) **EUROPEAN PATENT SPECIFICATION**

(45) Date of publication and mention
of the grant of the patent:
**11.08.2021 Bulletin 2021/32**

(21) Application number: **16741105.7**

(22) Date of filing: **10.06.2016**

(51) Int Cl.:
**A61M 15/00** *(2006.01)* **B05B 17/06** *(2006.01)*

(86) International application number:
**PCT/GB2016/051712**

(87) International publication number:
**WO 2016/198879 (15.12.2016 Gazette 2016/50)**

(54) **SPRAY DELIVERY DEVICE WITH AIR FLOW SENSING**

SPRÜHVORRICHTUNG MIT LUFTDURCHFLUSSSENSOR

DISPOSITIF DE PULVÉRISATION MUNI D'UN CAPTEUR DE DÉBIT D'AIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2015 GB 201510166**

(43) Date of publication of application:
**18.04.2018 Bulletin 2018/16**

(73) Proprietor: **TTP PLC**
**Melbourn,**
**Hertfordshire SG8 6EE (GB)**

(72) Inventor: **SELBY, Robert Gordon Maurice**
**Melbourn**
**Hertfordshire SG8 6EE (GB)**

(74) Representative: **Edson, Russell Gregory**
**Withers & Rogers LLP**
**4 More London Riverside**
**London SE1 2AU (GB)**

(56) References cited:
**EP-A1- 2 724 741       WO-A1-2013/098334**
**WO-A2-2009/102976       US-A- 5 694 919**

## Description

Field of the invention

This invention relates to electronic spray devices in which a perforate membrane is vibrated to generate liquid droplets. In particular, the invention relates to methods for enabling a delivery rate of the device to be tailored to the required rate, by reference to a demand signal from a suitable sensor.

Background

For inhalation devices such as electronic cigarettes the need for sensing flow to trigger smoke or droplet generation is well known. For example US2012186594 describes using a sensor to sense both the flow direction and rate of inhaled air. This sensor signal is used to determine when the air flow is in the right direction and exceeds a set threshold rate so as to trigger smoke generation.

There is a benefit in matching the delivery rate of any fluid, or medicament, to be delivered to a user, to suit the inhalation rate to provide a better user experience and also to maximise the efficiency of medicament/inhalant delivery. Similarly, for coating processes where droplet delivery is used to coat substrates, matching the delivery rate to a sensed property of the receiving substrate can optimise the quality of the coating and minimise wastage of the coating material. Furthermore when treating gas streams other than inhaled air matching the delivery rate to suit the gas flow rate will minimise cost and maximise benefits of the treating process if a sensor is used to determine the required delivery rate.

Electronic droplet generators that use ultrasonic vibration to generate liquid droplets are well known and have found use in a wide range of fields including medical drug delivery and the treatment of air or gas (for example fragrance delivery and humidification) and for coating of surfaces such as application of treatments to skin or industrial processes such as painting or applying surface treatments to components.

A preferred embodiment of such ultrasonic spray devices is one in which the perforate membrane itself is vibrated by the driver element (commonly called the actuator or vibrator element) with examples including US 4,533,082 and EP 0431992. This enables the delivery of relatively well monodispersed droplets without requiring the pressure waves to be transmitted through a liquid layer. A preferred embodiment of such a device is described in US 5,518,179 uses a bending mode actuator to deliver the vibrational energy to the membrane as this enables the use of thin low cost actuators.

In another example, US 5,694,919 provides a method for controlled access to a drug or medication using a microprocessor controlled locking system in combination with a handheld drug delivery device. The drug delivery device includes a valve or a springloaded plate which is actuated to release a drug formulation and may further include a vibrating porous membrane to assist with aerosol formation. The microprocessor prevents use of the device until a user ID has been confirmed and can optimise the timing of drug release for consistent delivery based on an airflow rate as detected by a flow sensor.

WO 2013/098334 and EP 2724741 each provide an inhalation device for drug delivery in which an aerosol may be generated by a vibrating mesh from a liquid formulation and which includes a feedback system configured to indicate to a user, by means of a signal during an inhalation event, whether an inhalation parameter, such as inspiratory flow rate, is within a target range.

Electronic spray technologies by definition require a power source and electronic circuitry (henceforth referred to as a spray controller) to be incorporated or linked to the spray generator.

Spray generators such as those described above often have a resonant frequency at which energy is efficiently transferred to the perforate membrane and hence to the liquid. To obtain good performance it is known that the spray generator must be operated at or at least close to the resonant frequency (EP 1,731,228 for example).

For such devices, the vibration is often generated by applying an alternating voltage across a unimorph or bimorph piezoceramic component or similar. The alternating voltage drives this component into oscillatory deformation at the drive frequency. This deformation is coupled to the perforate membrane causing it to vibrate and generate the liquid spray. Thus the characteristics of the input electrical waveform have a direct bearing on the spray that is generated.

WO 2009/102976 provides an aerosol therapy device in which aerosol is delivered through a nose piece in a pulsated airflow. The device includes an aerosol generator and a mouth piece through which the user can blow to trigger the operation of the aerosol generator and thereby cause the flow of aerosol through the nose piece.

Summary of the Invention

The invention provides a device for delivering a fluid spray to a mouth of a user, either directly, or via a flow conductor such as a tube or pipe. The spray device uses a vibrating perforate membrane to create the spray and the vibration of the membrane is controlled in response to input from a flow rate sensor which detects a flow rate through the device or through the flow conductor. The invention can be realised using the following features, any or all of which can be implemented in any combination to achieve the desired effects. The invention further provides a corresponding method of controlling a spray head in a spray delivery device.

According to the invention there is provided a spray delivery device for delivering a fluid spray to a fluid flow conductor, comprising:

a spray generator,

a spray controller; and

an airflow sensor;

wherein the spray generator comprises a perforate membrane and actuation means configured to ultrasonically vibrate the perforate membrane in response to a drive signal from the spray controller, such that vibration of the perforate membrane causes liquid droplets to be ejected from an ejection side of the perforate membrane;

characterised in that the flow sensor is configured to provide a flow signal representative of an air flow rate through the flow conductor, and

the spray controller is configured to control a spray rate of the spray generator in proportion to the air flow rate by modulation of the drive signal in response to the flow signal.

The flow signal may be proportional to the air flow rate or may be proportional to a function of the air flow rate.

The spray controller may be configured to generate no spray when the sensed air flow is below a predetermined threshold value.

The spray controller may be configured to modulate the drive signal using time based modulation.

The spray controller may be configured to modulate the drive signal by amplitude based modulation.

The spray controller may be configured to modulate the drive signal by shifting a frequency of the drive signal away from the resonant frequency of the device, such that for the same drive signal amplitude, a lower power is delivered by the spray generator.

The spray controller may be configured to modulate the drive signal by adjusting the mark space ratio of the drive signal.

The spray controller may be configured to adjust the mark space ratio of the drive signal such that the spray head drive is switched on and off at a frequency different from the resonant drive frequency.

The spray controller may be configured to adjust the mark space ratio of the drive signal such that the spray head drive is switched on and off at a frequency lower than the resonant drive frequency.

The spray controller may be configured to adjust the mark space ratio of the drive signal such that the spray head drive is switched on and off at a sufficient rate to provide at least 5 switching cycles within a 2 second time period.

The spray controller may be configured to multiply the flow signal by a proportionality constant, k, to derive the drive signal amplitude.

The device may be configured to permit a user to change the proportionality constant, k.

The controller may be configured to adjust the proportionality constant k over time to reduce the rate of dose delivery for a given inhalation rate over time.

The device may be configured to adapt the proportionality constant, k in response to the flow signal.

The device may be configured to adapt the proportionality constant, k, over a plurality of uses of the device, to adapt the delivery profile to suit the user's inhalation.

The device may be configured to adapt the proportionality constant, k, such that a defined dose is delivered substantially evenly within the user's typical inhalation time.

The device may be configured to adapt the proportionality constant, k, such that a defined dose is delivered substantially evenly within a predefined sub-portion of the user's total inhalation time.

The invention further provides an inhalation device comprising a spray delivery device according to any of the preceding claims, wherein the spray delivery device is configured to deliver the fluid spray to a fluid flow path through a body of the inhalation device.

The inhalation device may be configured for delivering nicotine based formulations or may be an electronic cigarette.

The invention further provides a device for delivering a fluid spray to a mouth of a user, either directly, or via a flow conductor such as a tube or pipe, the device comprising a vibratable perforate membrane for generating the spray, wherein the device is configured to control the vibration of the membrane by modulating a drive signal to the membrane in response to input from a flow rate sensor which detects a flow rate through the device or through the flow conductor.

The invention further provides a method of controlling a spray head in a spray delivery device, the spray head comprising a vibratable perforate membrane for generating a spray, the method comprising receiving a flow signal representative of an air flow rate through a flow conductor to which the spray head is arranged to deliver a spray, and controlling a spray rate of the spray head by modulating a drive signal to the vibratable perforate membrane in response to the flow signal.

Brief Description of the Figures

Embodiments of the invention will now be described with reference to the following figures in which:

Figure 1A shows a schematic representation of a device according to an embodiment of the present invention;
Figure 1B shows a schematic representation of a device according to a further embodiment of the present invention;
Figure 2 illustrates a first sensor signal and corresponding membrane drive signal for implementation in an embodiment of the invention;

Figure 3 illustrates a second sensor signal and corresponding membrane drive signal for implementation in an embodiment of the invention;

Figure 4 illustrates a mark space moderator signal and sensor signal for implementation in an embodiment of the invention;

Figure 5 illustrates a further mark space moderator signal and sensor signal for implementation in an embodiment of the invention;

Figure 6 illustrates a sensor signal and corresponding drive signal for implementation in an embodiment of the invention; and

Figure 7 illustrates a further example of a sensor signal and a corresponding drive signal for implementation in an embodiment of the invention.

Detailed Description of Embodiments of the Invention

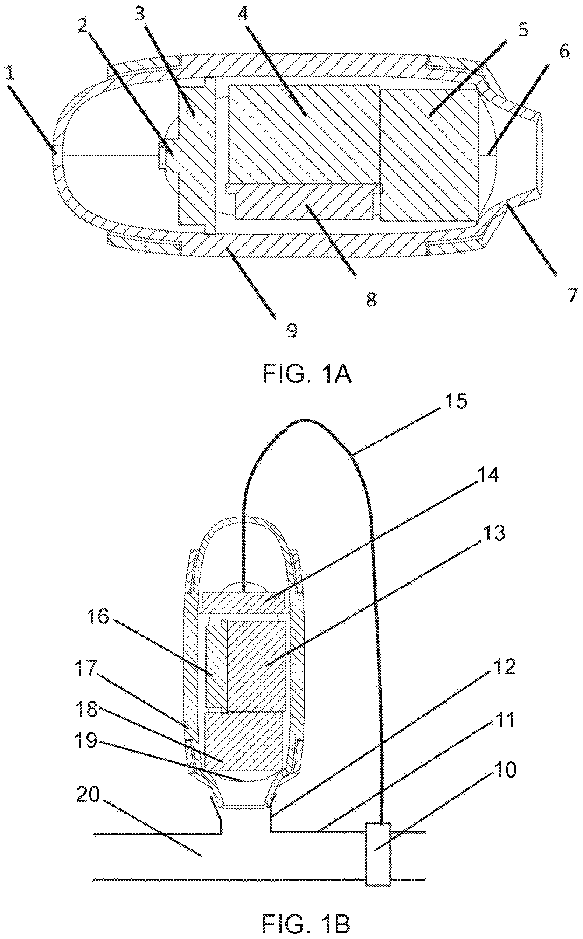

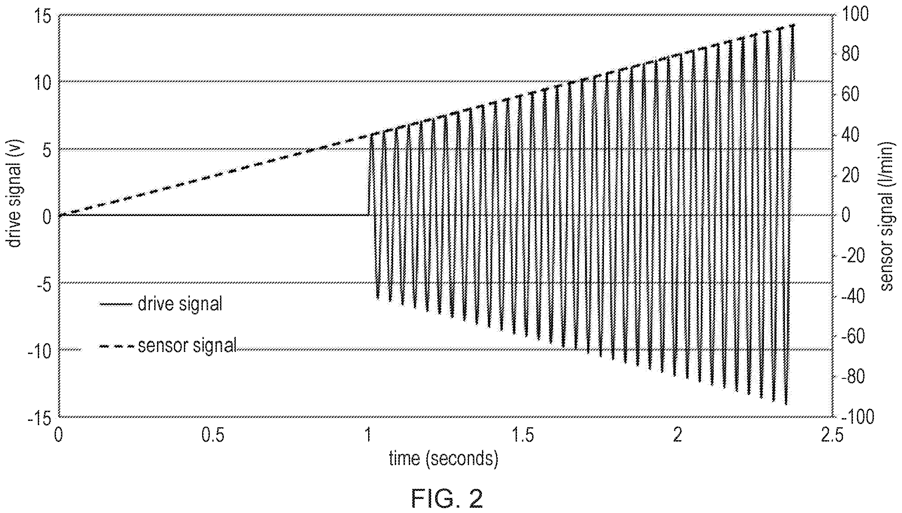

Figure 1A shows a schematic representation of an inhalation device according to an embodiment of the invention. The device has means to sense the inhaled airflow rate, preferably provided through the combination of the flow restrictor provide by opening (1) serving as a pressure restriction. The opening is typically of a cross sectional area of 6 to 12 mm$^2$ and the pressure sensor (2), preferably mounted on a PCB (3). The combination of the mouthpiece (7) and body (9) may form an otherwise sealed chamber with the exception of the opening (1) and the opening of the mouthpiece (7). When the user inhales the pressure drop induced by drawing air through the opening (1) is measured by the pressure sensor. The signal from the pressure sensor (2) is connected to and measured by the controller (microcontroller) (4). In turn the flow rate of air is calculated by the controller (4) and the appropriate delivery rate of fluid into the flow passing through the inhaler is determined for the measured inhalation rate. The controller then generates a drive signal to drive the atomising head (6). Variation of the drive signal allows adaption of the delivery rate to the inhalation rate by the means described in relation to figures 2 to 7. The controller (4) and PCB (3) are supplied with energy from the battery (8). The drive signal generated by the controller (4) is supplied to the perforate membrane based resonant atomising head (6) which in turn is mounted in a fluid reservoir (5). The resonant atomising head itself will typically consist of a PZT ceramic actuator bonded to a metallic substrate such that when the actuator is excited by an appropriate frequency alternating signal will vibrate and induce vibration in the perforate membrane.

An alternative configuration can be envisaged in which the spray delivery device is arranged adjacent a flow conductor such as a pipe, tube or other channel for conducting a fluid flow, preferably a gas flow such as air. The spray delivery device can be arranged to deliver spray into the fluid flow. A flow sensor can be configured to measure a flow in the flow conductor, and the spray device can be configured to deliver a fluid into the flow as a spray in response to the output of the flow sensor as described in the following. It will therefore be apparent that the delivery device and control means described herein can be used in an inhalation device or in a spray delivery device configured to deliver a spray into a flow conductor with which the spray device is placed in fluid communication.

An example of such an arrangement is shown in Figure 1B. In this figure the flow conductor (20) has a flow sensor (10) such as that produced by Honeywell S&C part number AWM720P1. This flow sensor provides a signal that is fed into the spray device (17) where the device, preferably on a daughter board (14), processes the flow signal to control the delivery of the spray. The drive PCB (13) and battery (16) in turn supply the necessary drive signal to the spray head (19), which delivers droplets at the appropriate rate from the fluid reservoir (18). The droplets are fed into the air stream in the flow conductor (20) via the port (12). Any of the drive methods discussed in the following can of course be used in either of the devices of Figures 1A and 1B.

Tuning the drive signal to the actuator can be achieved by a spray controller scanning a pre-programmed frequency band before commencing spraying, and using the results of this scan to record the resonant frequency of the spray generator and to use it as a later reference frequency. In this case the resonant frequency can be periodically checked by the controller whilst spraying so as to capture any shifts in resonant frequency due to changes in liquid loading for example. Alternatively a self-resonant drive circuit can be used in which the spray generator forms part of a resonant circuit such that the drive signal frequency is automatically tuned to the resonant frequency of the head.

The combination of an efficient and effective spray generator and drive electronics can allow high delivery rates to be achieved. This then offers the potential for tuning the fluid delivery rate to suit the specific and changing needs of the application. Typical applications that can benefit from adjusting the fluid flow or delivery rate in response to a variable parameter include: matching the fluid delivery rate to a flow rate of a gas stream, in the case of dosing the gas stream with an additional fluid, for example adding water droplets to allow control of humidity of a gas stream once the droplets evaporate; and adding anaesthetic agents to a gas stream in proportion to a flow rate of the gas. Further applications include drug delivery applications such as a nebuliser, inhaler or similar drug delivery device or consumer drug delivery device including nasal sprays and similar over the counter (OTC) devices where the delivery rate of a drug is moderated in proportion to the inhalation rate of the user.

For drug delivery applications, the flow rate could be proportional to the inhalation rate, optionally subject to a minimum trigger level below which delivery is not started.

**Methods of adjusting the delivery rate**

The output from the drive electronics for an ultrasonic spray generator can be adjusted in a number of ways to modulate the delivery rate. These methods include changing the output voltage, changing the drive frequency to be slightly offset from resonance, and using pulse-width modulation, as some examples.

The amplitude of vibration of a resonant, but damped, device can be controlled by the level or amplitude of the drive signal. Therefore, reducing the output voltage has the effect of reducing the number of nozzles on the spray head which are vibrating with sufficient acceleration to generate droplets, and so this reduces the flow rate. This method can provide progressive control of the flow rate within some limits. For example, below a certain drive voltage the flow rate will reduce very dramatically, as very many fewer nozzles will vibrate sufficiently to emit droplets. However this method has the disadvantage of needing a means to control the output drive voltage, which will typically require additional electronic components.

Similarly the amplitude of vibration is affected by how close frequency of stimulation is to the resonant frequency of the device. Adjusting the drive frequency to be slightly off the resonant frequency, for example by being offset from the resonant frequency by amounts such as 500 Hz to a few KHz away from the true resonant frequency. By characterising the behaviour of the actuator the degree of flow rate attenuation for a given offset from resonance allows the control of flow rate to be achieved and can therefore provide a second way in which to control the vibration and hence flow rate from such spray generators. This method has the advantage of using largely the same electronic components as those needed for basic driving of the spray generator, rather than needing additional components to adjust the drive voltage.

In addition to modification of frequency and/or voltage, another way to impact spray performance is through the use of time-based modulation of the drive signal, which we shall call "duty cycling". For pressurised sprays in industrial environments, pulsing of the spray by turning a valve on and off rapidly is used to adjust flow rate by such time-based modulation. This is commonly referred to as pulse width modulation. In general, flow rate is linearly proportional to on-time, thus a reduction in duty cycle from 100% (constantly on) to 50% (on half the time) would approximately halve the flow rate. The controller of figure 1 can therefore be configured to implement such a time-based modulation of the drive signal for the device of figure 1.

It is non-obvious that this approach would work with an electronic spray, as there is no valve to switch and the drive signal oscillates at high frequency to drive the spray generation process. However, it has been demonstrated that time-based modulation of this drive signal can be used to adjust the average flow rate of an electronic spray device. This approach works by applying the high frequency drive signal in bursts with gaps of no, or reduced, signal in between. The acceptable frequency of switching and the limits of on and off periods depend on application.

For example, for consumer applications, and in electronic cigarettes in particular, perception is critical. For some uses the overall period of this drive regime (burst time plus gap time) must be short enough that the plume appears to be continuous; whereas in some applications a slightly staccato delivery, having distinguishable gaps between bursts, may be appealing. In the case of micro-processor controlled drive electronics, the control parameters are set in firmware and so can be changed to suit user preference. In this case the frequency of pulse switching could be changed or, for example, for an electronic cigarette type device, the scaling between inhalation rate and delivery rate could be adjusted to adapt the delivery rate to the inhalation preference for the user. In one example we can consider the fluid delivery rate as being proportional to the inhaled flow rate, i.e. to the sensed gas flow rate:

$$Delivery\ rate = k\ x\ inhalation\ rate$$

where k is the proportionality factor. If $k$ is increased, the dose rate, or fluid delivery rate, for a given inhalation rate will increase and vice versa. A similar technique could be used to gradually reduce the dose over time, through an inbuilt algorithm provided in the spray controller, by reducing the value of k so that the user's dose could be gradually reduced, either during a single inhalation, or over multiple inhalations over an extended period of time.

The appearance of continuous delivery generally requires the overall period of the duty cycle, or otherwise stated, the time between each instance of the modulation signal switching from off to on, to be less than approximately 30 milliseconds, more ideally, less than 15 milliseconds. For dosing applications, such as treating a gas stream in a pipe, for example, for humidification or anasethetic applications, the acceptable limits for switching can have longer on and off periods, as mixing within the gas stream will average out the concentration and, further, the delivery will not be directly visible to the user. Therefore, the appearance of the delivery is less important. Given that a typical breath may be 2 seconds long, to provide the effect of proportional control, the rate at which changes can be made to the delivery rate is important. With a mark space ratio switching period of 15 milliseconds, over 100 periods of switching are available, giving a sufficiently high temporal resolution of on-off switching to give the impression of continuous variability. In this way, the speed of response of the ultrasonic spray device allows almost real time matching of dosing requirements to the demand signal from the sensor. The fluid delivery rate can also be adapted such that the total on-time within the user's typical inhalation time is sufficient to deliver a specific dose. The drive sequence for modulating the drive signal to create the desired fluid delivery rate can be iteratively adapted over a sequence of inhalation profiles. If the controller records the duration and total air volume of one or more successive inhalation events, the controller can establish a nominal inhalation duration and air volume for one or more of the recorded inhalation events. This can be based on the latest previous inhalation, or on a moving average of a number of previous inhalation events. Using this nominal inhalation duration, the controller can determine a proportionality constant K, such that the rate of delivery is tuned to be completed within the expected inhalation event. For example if the user typically inhales 2 litres of air in a 2 second inhalation and the set dose volume is 15 microlitres then an appropriate K value can be calculated. For simplicity if the inhalation rate is assumed to be constant for the two seconds and the delivery rate is similarly assumed to be constant for the delivery duration the delivery rate would be 7.5 microlitres per second and :

$$k = \frac{average\ inhalation\ rate}{delivery\ rate}$$

So the appropriate K value would be 0.125 microlitres per seconds per litre per minute of inhaled air.

By setting the K values in this way the controller can tune the actual delivery rate to suit the inhalation rate which will in practice vary through the inhalation. Increasing K by a few percent will ensure that the dose is delivered before the end of the inhalation. By successively adjusting the K value in this way over inhalation events allows tuning of the delivery to the anticipated inhalation profile for the user. This can all act to refine the delivery profile, such that the dose is delivered in a desired manner during a typical inhalation of a user. The chosen profile may be set to deliver the fluid evenly over the user's typical inhalation time. The profile may alternatively be adapted such that the fluid delivery is targeted for a particular sub-section of the user's typical inhalation, for example to ensure delivery to the deep lung by increasing the K value to ensure that the dose is delivered before the end of the inhalation event

In matching the required delivery rate to a measurable property, the performance of the overall system can be optimised. In the case of a drug delivery device, the dosing rate can be controlled to stay within tolerable limits or to optimise the delivery rate to suit the inhalation rate. This can be useful in the case of delivering a drug to patients with a low inspiration flow such as COPD [Chronic obstructive pulmonary disease] patients or children where the maximum delivery rate can be overwhelming. Similarly in self titration applications it has been found that users have a preference for the delivery rate to be in proportion to the inhalation rate. Expressed simply, the harder the user inhales the greater the delivery rate. The use of a pressure restriction and a pressure sensor can be used to create a measure of breath inhalation rate. For example, in an inhaler type device with a restriction providing a flow resistance of 0.2 $\frac{\sqrt{Mbar}}{l\ min}$, combined with a pressure sensor such as the amplified pressure sensor offered by Sensor Technics part number HDIM200DBE8H5, the inhalation rate can be measured and used to provide a signal to control the delivery rate.

The total dose volume can be controlled by the user controlling the inhalation duration and rate. Additionally or alternatively, a metering function can limit the maximum dose to be delivered before the device is re-primed by a user, or permitted to be re-primed, by the controller. In treating gas streams or setting the dose rate for a drug delivery device, an appropriate sensor such as a flow sensor may provide sufficient accuracy of demand signal. It may be further necessary to measure temperature and/or humidity to more fully characterise the target delivery rate.

Figure 2 illustrates an example of the modulation of a drive signal by the spray controller of the invention in response to a measured flow signal coming from the flow sensor of the device. The figure shows moderation of a sinusoidal drive signal, such as may be used for driving an ultrasonic actuator of the device of Figure 1, in response to a demand signal shown as a linearly rising signal. Typical drive frequencies for the ultrasonic spray generator may be in the range 30kHz to 200 kHz and more typically in the range 50 to 120 kHz. For clarity the drive signal is not shown with its real frequency in this figure.

Figure 2 also illustrates a deferred reaction to the demand signal, such that the drive signal is only initiated by the spray controller once the demand signal reaches a particular trigger threshold. In the case of inhalation through a drug delivery device typical value for the trigger threshold is 10 to 15 litres per minute. This serves as a good compromise between wanting the delivery to start early in the inhalation and providing a sufficiently robust sensor value to trigger, avoiding false triggering due to sensor signal noise or air movements due to factors other than inhalation occurring. Given that the time for inhalation rates to reach 10 or 15 litres per minute at the start of an inhalation is typically 0.1 seconds or sometimes shorter, this trigger level makes good use of the available inhaled volume. This has the advantage of avoiding false triggering events and also, in the case of amplitude modulation, prevents trying to drive the spray head with too low a voltage for effective operation.

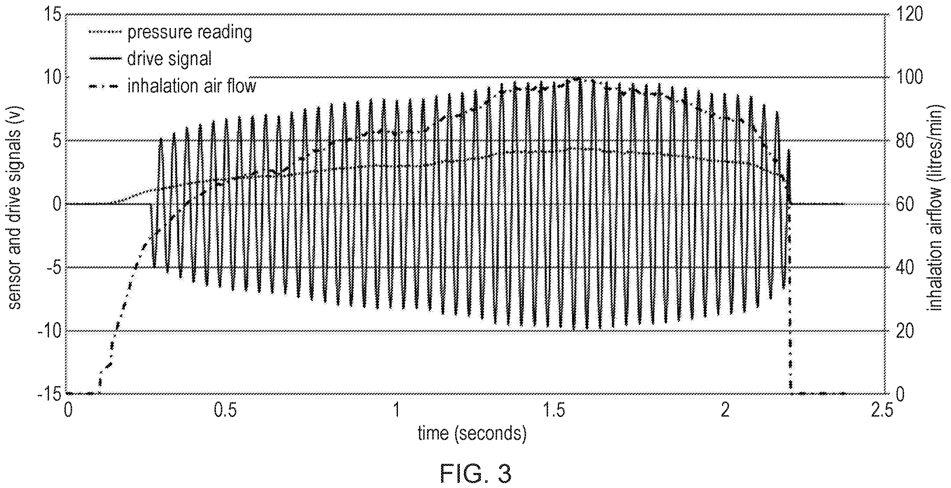

Figure 3 shows a similar arrangement, preferably where the demand signal is representative of an inhalation through a relatively high resistance drug delivery device, such as an inhaler or electronic cigarette. This implementation will tend to give a longer duration of an inhalation, such as the 2 seconds or thereabouts shown in the Figure. As in Figure 2, the drive signal is moderated in amplitude in proportion to the demand signal, which is the output of the flow sensor, and there is a defined minimum threshold trigger level, below which the drive signal is not initiated, which threshold may be at any of the values described above.

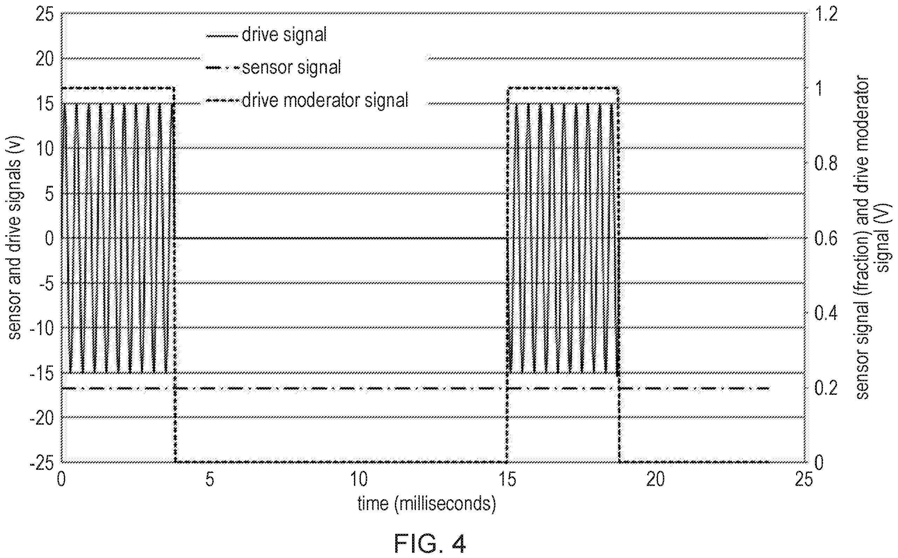

Figure 4 is an illustration of pulse width modulation of a drive waveform in response to a demand signal. The typical drive signal frequency may be 100 kHz and the period for the pulse width modulation may be in the range 5 to 30 ms. As such, the scales for these Figures have been adjusted to provide legibility and to illustrate the technique.

The graph of figure 4 shows the sensor or demand signal and the drive signal being on for approximately 4 ms and off for approximately 11 ms. This effects a 36% duty cycle in terms of both the time overwhich the spray generator is driven and in terms of the resulting time averaged fluid flow delivery rate.

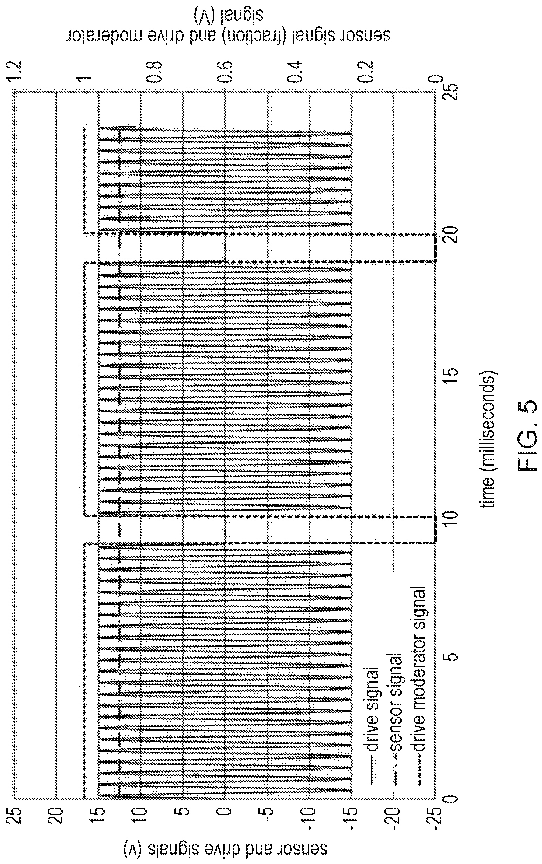

Figure 5 shows a similar arrangement, where the demand is higher and so the mark space ratio for the drive signal is higher. The graph shows the sensor or demand signal and the drive signal being on for approximately 8 ms and off for approximately 2 ms, an 80% duty cycle. Varying the duty cycle in this way has been shown to alter the delivery rate linearly with respect to the mark space ratio.

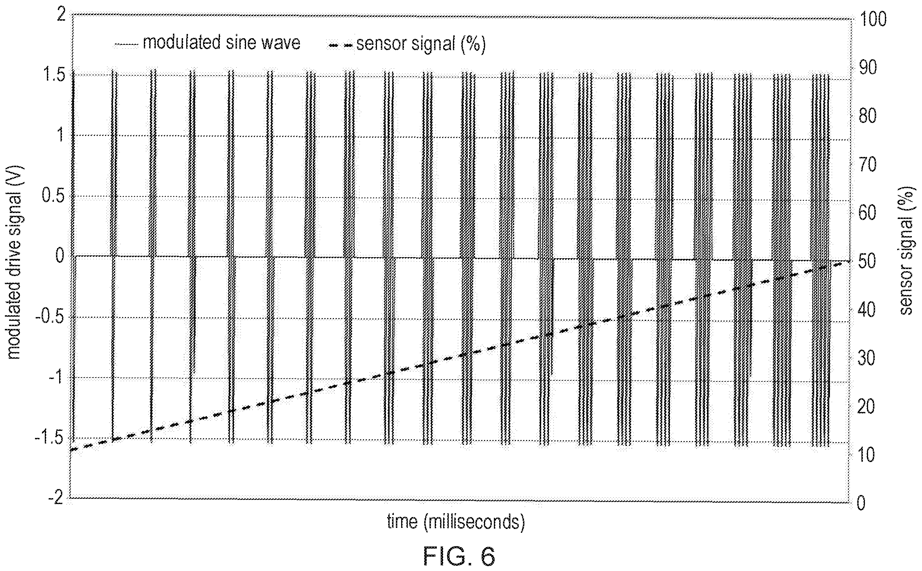

Figure 6 shows mark space modulation of the drive signal in response to a demand signal that is increasing linearly. The mark space ratio increases in a linear fashion in response to the rising sensor signal value and so matches the delivery rate to the demand.

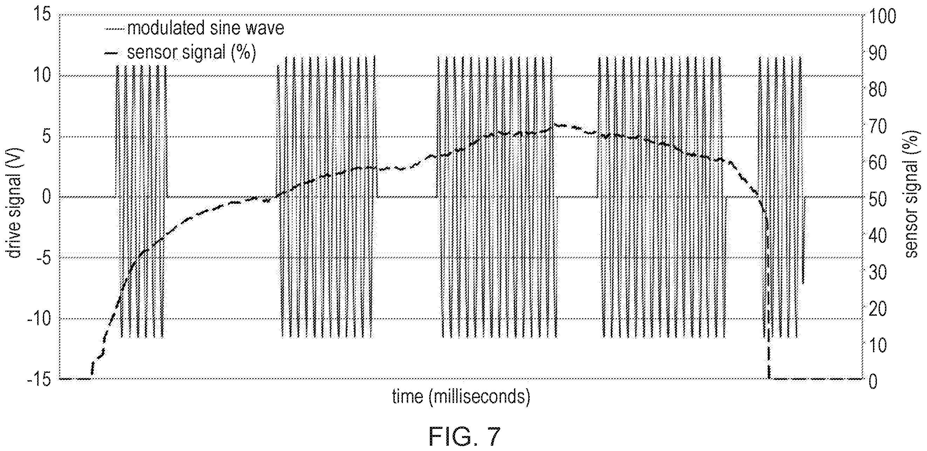

Figure 7 shows mark space modulation in response to inhalation breath profile signal, again periods of higher demand signal result in a higher mark space ratio and hence higher delivery rate.

In setting a mark space ratio for the figures 4 and 5 the inhaled air flow values may be in the range 0 to 100 litres per minute and if the controller is set to drive the head 100% of the time when the sensed air flow is 100 litres per second and proportionally across the range 0 to 100 litres per minutes e.g. for 50 litres per minute the duty cycle would be 50% and hence the mark space ratio would be set to 1:1 so on for 1 time unit and off for 1 time unit. The period of switching that set the duration of the on and off periods can be set to choice as discussed above, so typically for an inhalation device the switching period may be set to 15ms and hence a 1:1 mark space ratio would result in driving the head for 7.5ms and not driving the head for 7.5ms. Similarly for a 75 litre per minute air flow the mark space ratio would be 3:4 and the on time would be 11.25ms and the off time would be 3.75 ms within a switching period. In Figure 4 the flow signal is 0.25 (25%) of full scale value set for the controller. The switching period has been set to 15ms, so that the on time is 20% of 15 ms, so 3 ms, and the off time is 12 ms. In the example shown in Figure 5 the switching period has set at 10 ms and the sensed flow rate is 90% of the maximum expected values. Hence the on time is 9ms and the off time is 1ms.

Figure 6 shows the effect of the controller responding to a linearly increasing sensor signal (e.g. air flow). Again, the controller has been set to expect air flows up to 100 litres/minute and sets the mark space ratio in the proportion that the airflow rate is to the maximum values. By taking an average flow value within the switching period the controller calculated the mark space ratio appropriate for that switching period. So in the first switching period the flow average is 11 litres per minute (and 11% of maximum) so the mark space ratio is set to 11%. Consequently the on time is 1.1 ms and the off time is 8.9ms. At the right hand end of the graph the average flow rate is 50 litres per second (or 50% of the maximum expected value) and the on time is set to 5 ms and the off time is similarly 5ms.

In Figure 7 the configuration is similar to that of Figure 6. The average measured flow rate in a switching period is determined and this is used to set the mark space ratio. In the first switching cycle at the left hand side of the graph the average flow rate is 30% of the maximum and so the on time is 30 % of the switching period (3 ms) and the off time is 70% of the switching period (7 ms). In the 4th switching period the average measured flow is 80% of maximum and so the on time 8ms (80% of the switching period) and the off time is 2ms.

**Claims**

1. A spray delivery device for delivering a fluid spray to a fluid flow conductor (20), comprising:

    a spray generator (6, 19),
    a spray controller (4, 13); and
    an air flow sensor (2, 10);
    wherein the spray generator comprises a perforate membrane and actuation means configured to ultrasonically vibrate the perforate membrane in response to a drive signal from the spray controller, such that vibration of the perforate membrane is configured to cause liquid droplets to be ejected from an ejection side of the perforate membrane;
    **characterised in that** the airflow sensor is configured to provide a flow signal representative of an airflow rate through the fluid flow conductor, and
    the spray controller is configured to control a spray rate of the spray generator in proportion to the airflow rate by modulation of the drive signal in response to the flow signal.

2. A device according to claim 1, wherein the flow signal is proportional to the air flow rate or to a function of the air flow rate.

3. A device according to any of the preceding claims wherein the spray controller (4, 13) is configured to generate no spray when the sensed airflow is below a predetermined threshold value.

4. A device according to any of the preceding claims, wherein the spray controller (4, 13) is configured to modulate the drive signal using one or both of time based modulation and amplitude based modulation.

5. A device according to any of the preceding claims, wherein the spray controller (4, 13) is configured to modulate the drive signal by shifting a frequency of the drive signal away from the resonant frequency of the device, such that for the same drive signal amplitude, a lower power is delivered by the spray generator (6, 19).

6. A device according to any of the preceding claims, wherein the spray controller (4, 13) is configured to modulate the drive signal by adjusting the mark space ratio of the drive signal.

7. A device according to claim 6, wherein the spray controller (4, 13) is configured to adjust the mark space ratio of the drive signal such that the spray generator (6, 19) is switched on and off at a frequency different from, and preferably lower than, the resonant drive frequency.

8. A device according to claim 6 or claim 7, wherein the spray controller (4, 13) is configured to adjust the mark space ratio of the drive signal such that the spray generator (6,19) is switched on and off at a sufficient rate to provide at least 5 switching cycles within a 2 second time period.

9. A device according to any of the preceding claims, wherein the spray controller (4, 13) is configured to multiply the flow signal by a proportionality constant, k, to derive the drive signal amplitude.

10. A device according to claim 9, wherein the device is configured to permit a user to change the proportionality constant, k.

11. A device according to any of claims 9 or 10, wherein the spray controller (4, 13) is configured to adjust the proportionality constant k over time to reduce the rate of dose delivery for a given inhalation rate over time.

12. A device according to any of claims 9 to 11, wherein the device is configured to adapt the proportionality constant, k in response to the flow signal.

13. A device according to claim 12, wherein the device is configured to adapt the proportionality constant, k, over a plurality of uses of the device, to adapt the delivery profile to suit the user's inhalation.

14. A device according to claim 13, wherein the device is configured to adapt the proportionality constant, k, such that a defined dose is delivered substantially evenly within the user's typical inhalation time, preferably substantially evenly within a predefined subportion of the user's total inhalation time.

15. An inhalation device comprising a spray delivery device according to any of the preceding claims, wherein the spray delivery device is configured to deliver the fluid spray to a fluid flow path through a body (9) of the inhalation device.

16. An inhalation device according to claim 15, wherein the inhalation device is configured for delivering nicotine based formulations.

17. A device for delivering a fluid spray to a mouth of a user, either directly, or via a flow conductor (20) such as a tube or pipe, the device comprising a vibratable perforate membrane for generating the spray, **characterised in that** the device is configured to control the vibration of the membrane by modulating a drive signal to the membrane in response to input from a flow rate sensor (2, 10) which is configured to detect a flow rate through the device or through the flow conductor to control a spray rate from the perforate membrane in proportion to the flow rate.

18. A method of controlling a spray head (6, 19) in a spray delivery device, the spray head (6, 19) comprising a vibratable perforate membrane for generating a spray, the method comprising receiving a flow signal representative of an airflow rate through a flow conductor (20) to which the spray head is arranged to deliver a spray, and controlling a spray rate of the spray head in proportion to the air flow rate by modulating a drive signal to the vibratable perforate membrane in response to the flow signal.

**Patentansprüche**

1. Sprühvorrichtung zur Abgabe eines Fluidsprühstrahls an einen Fluidströmungsleiter (20), umfassend:

einen Sprühstrahlgenerator (6, 19),
eine Sprühstrahlsteuerung (4, 13); und
einen Luftströmungssensor (2, 10);
wobei der Sprühstrahlgenerator eine perforierte Membran und ein Betätigungsmittel aufweist, das dafür ausgebildet ist, die perforierte Membran in Reaktion auf ein Ansteuersignal von der Sprühstrahlsteuerung durch Ultraschall in Vibration zu versetzen, so dass die Vibration der perforierten Membran den Ausstoß von Fluidtröpfchen von einer Ausstoßseite der perforierten Membran bewirkt;
**dadurch gekennzeichnet, dass** der Luftströmungssensor konfiguriert ist für die Lieferung eines Strömungssignals, das repräsentativ ist für eine Luftströmungsrate durch den Fluidströmungsleiter, und wobei der Sprühstrahlregler konfiguriert ist für die Steuerung einer Sprührate des Sprühstrahlgenerators im Verhältnis zur Luftströmungsrate durch Modulieren des Ansteuersignals in Reaktion auf das Strömungssignal.

2. Vorrichtung nach Anspruch 1, wobei das Strömungssignal proportional zur Luftströmungsrate oder zu einer Funktion der Luftströmungsrate ist.

3. Vorrichtung nach einem der vorangehenden Ansprüche, wobei die Sprühstrahlsteuerung (4, 13) derart konfiguriert ist, dass kein Sprühstrahl erzeugt wird, wenn die erfasste Luftströmungsrate unter einem vorgegebenen Schwellenwert liegt.

4. Vorrichtung nach einem der vorangehenden Ansprüche, wobei die Sprühstrahlsteuerung (4, 13) ausgebildet ist zum Modulieren des Ansteuersignals durch Anwendung sowohl einer zeitbasierten Modulation als auch einer amplitudenbasierten Modulation.

5. Vorrichtung nach einem der vorangehenden Ansprüche, wobei die Sprühstrahlsteuerung (4, 13) ausgebildet ist zum Modulieren des Ansteuersignals durch die Verschiebung einer Frequenz weg von der Resonanzfrequenz der Vorrichtung, so das der Sprühstrahlgenerator (6, 19) bei gleicher Amplitude des Ansteuersignals eine geringere Leistung liefert.

6. Vorrichtung nach einem der vorangehenden Ansprüche, wobei die Sprühstrahlsteuerung (4, 13) ausgebildet ist zum Modulieren des Ansteuersignals durch Einstellen des Tastverhältnisses des Ansteuersignals.

7. Vorrichtung nach Anspruch 6, wobei die Sprühstrahlsteuerung (4, 13) ausgebildet ist zum Einstellen des Tastverhältnisses des Ansteuersignals derart, dass der Sprühstrahlgenerator (6, 19) ein- und ausgeschaltet wird mit einer Frequenz, die sich von der resonanten Ansteuerfrequenz unterscheidet und vorzugsweise niedriger als diese ist.

8. Vorrichtung nach Anspruch 6 oder Anspruch 7, wobei die Sprühstrahlsteuerung (4, 13) ausgebildet ist zum Einstellen des Tastverhältnisses des Ansteuersignals derart, dass der Sprühstrahlgenerator (6, 19) mit ausreichender Geschwindigkeit ein- und ausgeschaltet wird, um mindestens 5 Schaltzyklen innerhalb eines Zeitraums von 2 Sekunden zu ermöglichen.

9. Vorrichtung nach einem der vorangehenden Ansprüche, wobei die Sprühstrahlsteuerung (4 13) ausgebildet ist zum Multiplizieren des Strömungssignals mit einer Proportionalitätskonstante, k, um die Ansteuersignalamplitude abzuleiten.

10. Vorrichtung nach Anspruch 9, wobei die Vorrichtung derart konfiguriert ist, dass sie einem Nutzer erlaubt, die Proportionalitätskonstante, k, zu ändern.

11. Vorrichtung nach einem der Ansprüche 9 oder 10, wobei die Sprühstrahlsteuerung (4, 13) ausgebildet ist zum Einstellen der Proportionalitätskonstante k über die Zeit, um die Dosisabgaberate für eine gegebene Inhalationsrate über die Zeit zu verringern.

12. Vorrichtung nach einem der Ansprüche 9 bis 11, wobei die Vorrichtung ausgebildet ist zum Anpassen der Proportionalitätskonstante k in Reaktion auf das Strömungssignal.

13. Vorrichtung nach Anspruch 12, wobei die Vorrichtung ausgebildet ist zum Anpassen der Proportionalitätskonstante k über eine Mehrzahl von Nutzungen der Vorrichtung, um das Abgabeprofil an die Inhalation des Nutzers anzupassen.

14. Vorrichtung nach Anspruch 13, wobei die Vorrichtung ausgebildet ist zum Anpassen der Proportionalitätskonstante k derart, dass eine definierte Dosis innerhalb der typischen Inhalationszeit des Nutzers im Wesentlichen gleichmäßig abgegeben wird, vorzugsweise im Wesentlichen gleichmäßig innerhalb eines vordefinierten Teilbereichs der gesamten Inhalationszeit des Nutzers.

15. Inhalationsvorrichtung umfassend eine Sprühstrahlabgabevorrichtung nach einem der vorangehenden Ansprüche, wobei die Sprühstrahlabgabevorrichtung konfiguriert ist für die Abgabe des Fluidsprühstrahls durch einen Körper (9) der Inhalationsvorrichtung an einen Fluidströmungsweg.

16. Inhalationsvorrichtung nach Anspruch 15, wobei die Inhalationsvorrichtung konfiguriert ist für die Abgabe von Formulierungen auf Nikotinbasis.

17. Vorrichtung zur Abgabe eines Fluidsprühstrahls in den Mund eines Nutzers entweder direkt oder über einen Strömungsleiter (20) wie ein Schlauch oder ein Rohr, wobei die Vorrichtung eine perforierte Membran aufweist, die zum Erzeugen des Sprühstrahls in Vibration versetzt werden kann, **dadurch gekennzeichnet, dass** die Vorrichtung ausgebildet ist zum Steuern der Vibration der Membran durch Modulieren eines Ansteuersignals zur Membran in Reaktion auf die Eingabe von einem Strömungssensor (2, 10), der konfiguriert ist für die Erfassung einer Strömungsrate durch die Vorrichtung oder durch den Strömungsleiter zum Steuern einer Sprühstrahlrate von der perforierten Membran im Verhältnis zur Strömungsrate.

18. Verfahren zum Steuern eines Sprühkopfs (6, 19) in einer Sprühstrahlabgabevorrichtung, wobei der Sprühkopf (6, 19) eine Membran zur Erzeugung eines Sprühstrahls aufweist, die in Vibration versetzt werden kann, wobei das Verfahren den Empfang eines Strömungssignals umfasst, das repräsentativ ist für Strömungsrate durch einen Strömungsleiter (20), an welchem der Sprühkopf für die Abgabe eines Sprühstrahls angeordnet ist, und das Steuern einer Sprührate des Sprühkopfs im Verhältnis zur Luftströmungsrate durch Modulieren eines Ansteuersignals zu der Membran, die in Vibration versetzt werden kann, in Reaktion auf das Strömungssignal.

**Revendications**

1. Dispositif de pulvérisation pour distribuer un liquide pulvérisé dans un conducteur de débit de fluide (20) comprenant :

   - un générateur de pulvérisation (6, 19),
   - un contrôleur de pulvérisation (4, 13) et,
   - un capteur de débit d'air (2, 10),

   selon lequel
   le générateur de pulvérisation comporte une membrane perforée et des moyens d'actionnement configurés pour faire vibrer par des ultrasons la membrane perforée en réponse à un signal de commande du contrôleur de pulvérisation, cette vibration de la membrane perforée étant configurée pour provoquer l'éjection de gouttelettes de liquide du côté éjection de la membrane perforée,
   dispositif **caractérisé en ce que**
   le capteur de débit d'air est configuré pour fournir un signal de débit représentant le débit d'air à travers le conducteur de flux de fluide et le contrôleur de pulvérisation est configuré pour commander le débit de pulvérisation du générateur de pulvérisation proportionnellement au débit d'air en modulant le signal de commande en réponse au signal de débit.

2. Dispositif selon la revendication 1, selon lequel
   le signal de débit est proportionnel au débit d'air ou est une fonction du débit d'air.

3. Dispositif selon l'une quelconque des revendications précédentes, selon lequel

la commande de pulvérisation (4, 13) est configurée pour ne pas générer de pulvérisation si le débit d'air détecté est inférieur à un seuil prédéterminé.

4. Dispositif selon l'une quelconque des revendications précédentes, selon lequel
   la commande de pulvérisation (4, 13) est configurée pour moduler le signal d'entraînement en utilisant l'une ou l'autre ou les deux modulations en fonction du temps et l'amplitude fondée sur la modulation.

5. Dispositif selon l'une quelconque des revendications précédentes, selon lequel
   la commande de pulvérisation (4, 13) est configurée pour moduler le signal d'entraînement en décalant la fréquence du signal d'entraînement par rapport à la fréquence de résonnance du dispositif de façon que pour la même amplitude de signal d'entraînement, le générateur de pulvérisation (6, 19) fournisse une puissance inférieure.

6. Dispositif selon l'une quelconque des revendications précédentes, selon lequel
   la commande de pulvérisation (4, 13) est configurée pour moduler le signal d'entraînement réglant le rapport cyclique du signal d'entraînement.

7. Dispositif selon la revendication 6, selon lequel
   la commande de pulvérisation (4, 13) est configurée pour régler le rapport cyclique du signal d'entraînement de façon que le générateur de pulvérisation (6, 19) commute en arrêt / marche à une fréquence différente de la fréquence de résonnance d'entraînement et de préférence, une fréquence inférieure à celle-ci.

8. Dispositif selon la revendication 6 ou la revendication 7, selon lequel
   la commande de pulvérisation (4, 13) est configurée pour régler le rapport cyclique du signal d'entraînement de façon que le générateur de pulvérisation (6, 19) commute en marche / arrêt à une vitesse suffisante pour avoir au moins 5 cycles de commutation dans une durée de 2 secondes.

9. Dispositif selon l'une quelconque des revendications précédentes, selon lequel
   la commande de pulvérisation (4, 13) est configurée pour multiplier le signal de débit avec une constante de proportionnalité k pour dériver l'amplitude du signal d'entraînement.

**10.** Dispositif selon la revendication 9, selon lequel le dispositif est configuré pour permettre à l'utilisateur de modifier la constante de proportionnalité k.

**11.** Dispositif selon l'une quelconque des revendications 9 ou 10, selon lequel la commande de pulvérisation (4, 13) est configurée pour régler la constante de proportionnalité k en fonction du temps pour réduire la vitesse de distribution dosée pour une vitesse d'inhalation donnée, en fonction du temps.

**12.** Dispositif selon l'une quelconque des revendications 9 à 11, selon lequel le dispositif est configuré pour adapter la constante de proportionnalité k en réponse au signal de débit.

**13.** Dispositif selon la revendication 12, selon lequel le dispositif est configuré pour adapter la constante de proportionnalité k pour un ensemble d'utilisations du dispositif, pour adapter le profil de distribution adapté à l'inhalation de l'utilisateur.

**14.** Dispositif selon la revendication 13, selon lequel le dispositif est configuré pour adapter la constante de proportionnalité k de façon qu'une dose définie soit délivrée pratiquement régulièrement pendant toute la durée d'inhalation caractéristique de l'utilisateur, de préférence pratiquement régulièrement dans une sous-partie prédéfinie du temps total d'inhalation de l'utilisateur.

**15.** Dispositif d'inhalation comprenant un distributeur de pulvérisation selon l'une quelconque des revendications précédentes, selon lequel le distributeur de pulvérisation est configuré pour distribuer le fluide pulvérisé vers un chemin de passage de fluide à travers le corps (9) du dispositif d'inhalation.

**16.** Dispositif d'inhalation selon la revendication 15, selon lequel le dispositif d'inhalation est configuré pour distribuer des compositions à base de nicotine.

**17.** Dispositif de distribution d'une pulvérisation dans la bouche d'un utilisateur soit directement, soit par l'intermédiaire d'un conducteur de flux (20) tel qu'un tube ou une conduite, le dispositif comportant une membrane perforée, vibrante, pour générer la pulvérisation, dispositif **caractérisé en ce qu'**il est configuré

pour commander la vibration de la membrane en modulant le signal d'entraînement de la membrane en réponse à une entrée d'un capteur de débit (2, 10) configuré pour détecter le débit dans le dispositif ou dans le conducteur de flux, pour commander la vitesse de pulvérisation à partir de la membrane perforée, proportionnellement au débit.

**18.** Procédé de commande d'une tête de pulvérisation (6, 19) d'un dispositif de pulvérisation, la tête de pulvérisation (6, 19) comportant une membrane perforée susceptible de vibrer pour générer une pulvérisation, le procédé consistant à recevoir le signal de débit représentant le débit d'air à travers un conducteur de flux (20) qui est équipé de la tête de pulvérisation organisée pour distribuer une pulvérisation et commander un débit de pulvérisation de la tête de pulvérisation, proportionnel au débit d'air en modulant avec le signal d'entraînement de la membrane perforée, vibrant en réponse au signal de débit.

FIG. 1A

FIG. 1B

FIG. 2

FIG. 3

FIG. 4

FIG. 5

FIG. 6

FIG. 7

**EP 3 307 366 B1**

**Patent documents cited in the description**

- US 2012186594 A
- US 4533082 A
- EP 0431992 A
- US 5518179 A
- US 5694919 A
- WO 2013098334 A
- EP 2724741 A
- EP 1731228 A
- WO 2009102976 A